Europäisches Patentamt

European Patent Office    (11) Numéro de publication : **0 091 876**

Office européen des brevets    **B1**

(19)

(12)    # FASCICULE DE BREVET EUROPÉEN

(45) Date de publication du fascicule du brevet :
02.03.88

(51) Int. Cl.⁴ : **A 61 C 5/10**, A 61 C 9/00,
A 61 C 19/04

(21) Numéro de dépôt : 83420065.1

(22) Date de dépôt : 14.04.83

(54) Dispositif de prise d'empreinte par des moyens optiques, notamment en vue de la réalisation automatique de prothèses.

(30) Priorité : 14.04.82 FR 8206707

(43) Date de publication de la demande :
19.10.83 Bulletin 83/42

(45) Mention de la délivrance du brevet :
02.03.88 Bulletin 88/09

(84) Etats contractants désignés :
AT BE CH DE FR GB IT LI LU NL SE

(56) Documents cités :
EP-A- 0 025 911
EP-A- 0 033 492
EP-A- 0 040 165
EP-A- 0 054 785
FR-A- 2 121 483

(73) Titulaire : **Duret, Francois**
**Rue Paul Claudel**
**Le Grand Lemps Isère (FR)**

**Michallet, Elisabeth epouse Duret**
**Rue Paul Claudel**
**Le Grand Lemps Isère (FR)**

**Termoz, Christian**
**2, Place Victor Hugo**
**Grenoble Isère (FR)**

(72) Inventeur : **Duret, Francois**
**rue Paul Claudel**
**Le Grand Lemps, Isère (FR)**

(74) Mandataire : **Maureau, Philippe**
**Cabinet Germain & Maureau Le Britannia - Tour C 20,**
**bld Eugène Déruelle**
**F-69003 Lyon (FR)**

## Description

La présente invention se rapporte à un dispositif de prise d'empreinte par des moyens optiques, notamment en vue de la réalisation automatique d'une prothèse, et encore plus particulièrement la réalisation de prothèses telles que les couronnes utilisées en art dentaire, ce dispositif comprenant, en combinaison, des moyens émetteurs d'ondes lumineuses non traumatisantes ; une tête d'analyse incluant des moyens transmetteurs pour diriger lesdites ondes sur la partie du corps, telle qu'emplacement de dent, à analyser, et des moyens récepteurs des ondes lumineuses réfléchies par cette partie du corps, dirigeant lesdites ondes sur un capteur, ledit capteur étant associé à un convertisseur analogique-numérique, permettant d'obtenir sous forme de signaux numériques une représentation de la forme, dans les trois dimensions de l'espace, de la partie du corps à analyser, et des moyens d'analyse et de traitement des signaux numériques obtenus, notamment en vue de la commande automatique d'une machine-outil à commande numérique pour l'usinage d'une prothèse, telle qu'une prothèse dentaire adaptée exactement à la partie du corps analysée (selon document EP-A-0 040 165). Cette application n'est toutefois pas limitative, en ce sens que le dispositif est aussi utilisable pour l'établissement d'un diagnostic odontologique qui n'est pas suivi de la confection d'une prothèse.

Très peu d'études ont, jusqu'à présent, eu pour objet la prise d'une empreinte par des moyens optiques, dans le domaine de l'art dentaire, et les réalisations concrètes sont actuellement inexistantes. On peut citer le brevet US-A-3 861 044 (SWINSON), document décrivant un procédé qui consiste, pour faire l'empreinte d'une cavité creusée dans une dent et réaliser un insert (ou « inlay ») destiné à se loger dans la cavité, en la séquence d'opérations suivantes :

— préparation de la dent défectueuse ;

— réalisation d'une représentation photographique de la dent avec sa cavité ;

— transfert d'un signal représentatif de l'image photographiée à une machine-outil commandée automatiquement ;

— remplissage de la dent préparée avec de la cire ;

— réalisation d'une nouvelle représentation photographique de la dent, cette fois remplie de cire ;

— transfert d'un signal représentatif de la nouvelle image photographique à la machine-outil commandée automatiquement ;

— fonctionnement automatique de la machine-outil à partir des signaux précédents, afin d'usiner un insert dentaire de forme adaptée ;

— mise en place de l'insert dans la cavité de la dent.

Cette technique arbitraire, telle qu'elle est décrite dans le document auquel il est ici fait référence, présente de nombreux inconvénients ainsi que des insuffisances diverses :

1. Une image photographique ordinaire ne peut représenter qu'en deux dimensions l'objet dont la forme est à analyser. Si l'on veut reconstituer par ce procédé les trois dimensions de l'espace, il faut réaliser un grand nombre de photographies, par exemple de l'ordre du millier au minimum, avec une précision de 100 μm, pour chaque élément à analyser.

2. Il n'est pas précisé quelle est la nature des signaux transmis, supposés représentatifs des images photographiques réalisées, et en particulier si ces signaux sont analogiques ou numériques. Or un usignage automatique ne peut se faire qu'au moyen d'une machine-outil à commande numérique, et dans le cas considéré l'on ne voit donc pas concrètement comment la machine pourra effectuer un travail déterminé, ni surtout un positionnement strict de la pièce à usiner, à partir d'images photographiques traduites en des signaux de nature indéfinie.

3. Dans le procédé évoqué, le praticien doit remplir la cavité de cire. Cette technique est possible pour les inserts, mais inapplicable à d'autres types courants de prothèses pour dents, notamment les couronnes, ce qui exclut bien évidemment ce genre d'applications.

4. De plus, le remplissage de cire et son utilisation constituent une sorte de prise d'empreinte par moulage ; le procédé exige ainsi des opérations matérielles sur la dent à traiter et ne peut s'assimiler à une prise d'empreinte complète par des moyens purement optiques ; le temps de mise en œuvre reste donc également important. Enfin, le second signal photographique, obtenu à partir de ce remplissage de cire, est toujours aussi imprécis (reconstitution des trois dimensions ?), et cette imprécision se répercute évidemment sur le résultat final obtenu.

Le document EP-A-0 025 911 propose un système de fabrication de prothèses dentaires, permettant la confection partiellement automatique d'une prothèse, avec prise d'empreinte optique basée sur le principe de la photogrammétrie. Ce système ne permet toutefois pas la fabrication directe et entièrement automatique de la prothèse dentaire, car il implique deux saisies optiques successives et une opération manuelle, le processus complet étant en résumé le suivant :

1. Confection automatique d'un « modèle de travail » du moignon de dent, par une première analyse optique.

2. Opération manuelle de confection d'un « modèle de travail » de la prothèse.

3. Reproduction automatique de ce modèle de la prothèse.

De plus, le procédé photogrammétrique du document EP-A-0 025 911 exige un temps de mise en œuvre très long, et il paraît inapplicable à l'analyse d'un objet situé en bouche, pour laquelle il faut tenir compte des mouvements inévitables du patient, et pour laquelle il se pose aussi des problèmes de repérage, l'analyse optique ne pouvant se faire sans points de référence et sans possibilité de corrélation des surfaces.

Le document EP-A-0 040 165 fournit déjà les grandes lignes d'un procédé permettant d'analyser en trois dimensions la forme d'un moignon de dent, en fournissant un signal directement utilisable par une machine-outil à commande numérique pour la confection automatique et directe d'une prothèse adaptable sur le moignon de dent, sans aucune intervention intermédiaire ni en bouche ni sur un « modèle de travail ». Ce procédé fait essentiellement appel à la technique holographique avec projection d'une onde monochromatique de type laser, non traumatisante, et utilisation d'un capteur et d'un convertisseur analogique-numérique correspondant au type d'ondes mis en œuvre.

Si l'on envisage dans un procédé analogue l'utilisation de lumière non cohérente qui paraît intéressante pour l'analyse optique, il se pose le problème, déjà évoqué plus haut à propos du document EP-A-0 025 911, du repérage par rapport à une référence.

Ce problème est résolu, selon l'invention, par un dispositif de prise d'empreinte par des moyens optiques, notamment en vue de la réalisation automatique de prothèses, appartenant au genre indiqué en introduction, dans lequel lesdits moyens émetteurs comprennent une source émettrice de lumière non cohérente, ce dispositif comprenant en outre, pour permettre l'analyse par interférométrie holographique, des trames de repérage (connues en soi) associées respectivement à la source émettrice et au capteur, et des moyens de détermination de distance permettant de déterminer ou de fixer la distance entre les moyens optiques de la tête d'analyse et un plan de référence lié à la partie du corps à analyser. Selon une première solution, dite « dynamique », les moyens de détermination de distance associés à la tête d'analyse sont constitués par un émetteur-récepteur d'ultra-sons ou d'infra-rouge déterminant la distance de ladite tête d'analyse par rapport au plan de référence au moment précis où est réalisé l'éclairage par la source. Selon une seconde solution, dite « statique », les moyens de détermination de distance associés à la tête d'analyse sont constitués par un repère de longueur connue, destiné à reposer sur un point de la partie du corps à analyser.

Des systèmes de miroirs, et des optiques plus ou moins « larges » permettent l'analyse de zones plus ou moins étendues, pouvant posséder des faces orientées différemment les unes des autres, ou des faces qui ne peuvent être directement éclairées et/ou observées.

Le capteur, atteint par le faisceau réfléchi et aussi éventuellement par un faisceau de référence, est avantageusement du type photosenseur à transfert de charge, notamment un photosenseur du type CCD (Charge Coupled Devices) matriciel, ou encore un tube vidicon modifié.

L'intérêt du photosenseur CCD sur le système vidicon est qu'il fournit une analyse plan par plan ; par contre le nombre de niveaux de gris y est inférieur, actuellement. Afin de ne pas surcharger les moyens de traitement des données, l'analyse se fait en fixant les coordonnées suivant deux dimensions, par exemple en des points correspondant à des intervalles de 20 $\mu$m, et en ne faisant varier que les coordonnées suivant la troisième dimension de l'espace.

Suivant un mode de réalisation, des moyens de stockage de l'information sont insérés entre le capteur et le convertisseur analogique-numérique proprement dit, lequel délivre vers les moyens de traitement des signaux de sortie « point par point », transformés par une interface pour leur adaptation aux moyens de traitement numérique, des moyens de visualisation des données saisies étant en outre prévus, en liaison avec les moyens de stockage précités.

La visualisation doit être de préférence prévue, pour information et vérification, afin de pouvoir apprécier si l'empreinte optique réalisée est totale, précise, exploitable et prise sur un travail préparatoire (taille d'un moignon de dent) effectué correctement. Plus particulièrement la visualisation est avantageuse avant conversion analogique-numérique, afin de limiter le champ d'action (suppression des informations jugées inutiles) et vérification de la qualité de la saisie des données. La visualisation de l'image sous sa forme obtenue après conversion analogique-numérique est aussi utile, pour apprécier ligne par ligne la précision des données. Le choix de la meilleure image, à retenir pour le traitement numérique ultérieur, pourra être manuel et facilité par une visualisation interactive, ou même être entièrement automatique.

Le traitement numérique, préalable à la réalisation d'une prothèse, doit dans le cas pris ici pour exemple d'une couronne dentaire tenir compte encore d'autres données que les seules caractéristiques géométriques du moignon de dent, déterminées comme résultat de l'analyse optique, et à cet effet les moyens d'analyse et de traitement comprennent encore, de préférence :

— des moyens de détermination et de prise en compte de l'enveloppe, ou volume à l'intérieur duquel doit s'inscrire la dent reconstituée ;

— des moyens de détermination et prise en compte de l'occlusion, statique et dynamique ;

— éventuellement, des moyens de détermination et de prise en compte des couleurs ou teintes de dents.

Plus particulièrement, l'enveloppe ou volume à l'intérieur duquel doit s'inscrire la prothèse à usiner est déterminée latéralement par :

a) les zones de contact des dents voisines ; et

b) les plans tangents à l'arcade ou déterminés sur le côté symétrique, pour les faces vestibulaires ou linguales ; inférieurement par :

c) les limites inférieures du moignon analysé en bouche, pour le plan inférieur ; et supérieurement par :

d) un plan supérieur en fonction d'une analyse mathématique issue soit de l'étude des zones d'usure de l'ensemble du maxillaire, soit du déplacement réel de la mandibule.

Les moyens d'analyse et traitement des signaux peuvent comprendre encore des moyens de correction automatique de la forme extérieure théorique de la prothèse inscrite dans l'enveloppe précitée, en fonction de données résultant de l'analyse optique, telles que la forme du moignon de dent dans le cas de réalisation d'une couronne.

Dans le cas d'application de l'invention à la confection automatique de prothèses mobiles, l'occlusion réelle pourra être déterminée non seulement par la prise d'empreinte optique mais encore par une analyse faciale et buccale du patient sur lequel on aura préalablement placé un certain nombre de repères buccaux, évitant une étude de l'articulation temporaux-maxillaire par le moyen classique, très coûteux, de l'articulateur.

L'occlusion pourra être déterminée par repérage des maxillaires supérieur et inférieur, prises d'empreinte optique séparées des deux maxillaires, réalisation d'une claie optique antérieure des repères des deux maxillaires, en réunion des deux maxillaires grâce à leurs repérages au niveau de la claie.

La réalisation automatique de la prothèse, telle que notamment couronne, passe par les étapes suivantes, exploitant les opérations d'analyse et de détermination diverses exposées précédemment :

1. Réalisation de la partie interne de la prothèse, en fonction essentiellement de l'empreinte optique réalisée, avec modification éventuelle des données brutes selon le type de fixation (espace souhaité pour le ciment, ou fixation sans joint par élasticité du métal), selon la position de l'axe d'insertion, ou selon les imperfections non corrigées de la taille du moignon.

2. Adaptation d'une forme extérieure théorique, stockée en mémoire, à l'enveloppe précédemment déterminée, et ajustage de cette forme selon la détermination faite de l'occlusion.

3. Choix du mode d'usinage et du matériau, tenant compte entre autres de la détermination préalable de couleur ou teinte.

Les outils doivent évidemment être adaptés aux dimensions et aux courbures des pièces prothétiques à confectionner, dont l'ébauche devra être fixée rigidement de manière à éviter les vibrations et à fournir un point de référence lors de l'usinage. Les principes connus de programmation, de repérage, et d'asservissement pour l'utilisation des machines-outils à commande numérique s'appliqueront sans difficultés, l'usinage s'effectuant de préférence sous contrôle visuel de ses différentes phases par les moyens de visualisation mentionnés plus haut.

Pour une précision recherchée de 50 microns, l'on peut estimer à 5 minutes le temps nécessaire à l'usinage d'une couronne ; la réalisation complète d'une couronne, incluant la prise d'empreinte optique, le traitement des données et l'usinage lui-même, ne devrait pas dépasser une durée totale de 15 minutes, alors que le temps nécessaire est actuellement de l'ordre d'une semaine, avec plus de 3 heures de travail effectif de dentiste et de prothésiste, en se référant au procédé classique de la « cire perdue ». Le temps réel de travail est donc divisé par 12, et le temps séparant la prise d'empreinte de la pose de la prothèse est divisé par 600, tout en améliorant les conditions biologiques et physiologiques de l'opération puisque le patient pourra repartir avec sa dent reconstituée 15 minutes après la fin de la taille.

De toute façon, l'invention sera mieux comprise à l'aide de la description qui suit, en référence au dessin schématique annexé représentant, à titre d'exemples non limitatifs, quelques formes d'exécution de ce dispositif de prise d'empreinte, notamment en vue de la réalisation de prothèses, et illustrant diverses applications de ce dispositif :

Figure 1 est une vue très schématique d'une première forme de réalisation de la partie optique du dispositif selon l'invention ;

Figure 2 montre un détail de la tête d'analyse optique, dans un mode de réalisation particulier ;

Figure 3 est une vue d'ensemble des moyens permettant la prise d'empreinte optique, selon le principe de la figure 1, dans un agencement particulier ;

Figure 4 est un schéma de principe de la partie optique du dispositif selon l'invention ;

Figures 5, 6 et 7 sont des vues d'ensemble des moyens permettant la prise d'empreinte optique, dans divers agencements ;

Figures 8 et 9 sont des schémas de systèmes optiques de prise d'empreinte, utilisant en outre des miroirs ;

Figure 10 montre le système optique du dispositif selon l'invention, appliqué à l'analyse des arcades ;

Figures 11, 12 et 13 sont des vues de détail du système optique de figure 10, muni de divers moyens d'appui ;

Figure 14 est un schéma-bloc des moyens de réception des ondes réfléchies, de conversion analogique-numérique et de visualisation interactive du dispositif, pour l'obtention de l'empreinte optique traduite numériquement, dans le cas d'utilisation d'un capteur du type « photosenseur CCD » ;

Figure 15 est une vue en plan illustrant la détermination de plusieurs des plans définissant l'enveloppe d'une couronne à réaliser ;

Figure 16 montre le principe d'adaptation de la forme extérieure d'une couronne, déterminée théoriquement, à la forme et à la position du moignon analysées par la prise d'empreinte optique ;

Figure 17 illustre, dans le cas d'application à une prothèse mobile, la définition des plans et surfaces d'enveloppe ;

Figure 18 est un schéma expliquant le positionnement relatif des empreintes supérieure et inférieure intervenant dans la détermination de l'occlusion réelle ;

Figure 19 est un schéma relatif à la détermination de l'occlusion ;

Figure 20 est un schéma relatif à l'exploitation de repères et autres capteurs, pour la prise en compte de la pathologie de l'articulation temporaux-maxillaire ;

Figure 21 est un schéma relatif à la définition des caractéristiques d'une prothèse dentaire fixe, à sa réalisation et à son adaptation, à partir de prises d'empreintes optiques ;

Figure 22 est un schéma relatif à la définition des caractéristiques d'une prothèse dentaire mobile, et à sa réalisation, dans le cas d'une prothèse avec antagoniste ;

Figure 23 est un schéma des fonctions complémentaires, à réaliser dans le cas d'une prothèse dentaire mobile sans antagoniste ;

Figure 24 est un schéma-bloc d'usinage automatique d'une prothèse et de contrôle d'usinage, par le dispositif objet de l'invention ;

Figure 25 montre les formes à usiner pour la réalisation d'une couronne ;

Figure 26 montre les formes à usiner pour la réalisation d'une prothèse avec pivot de fixation ;

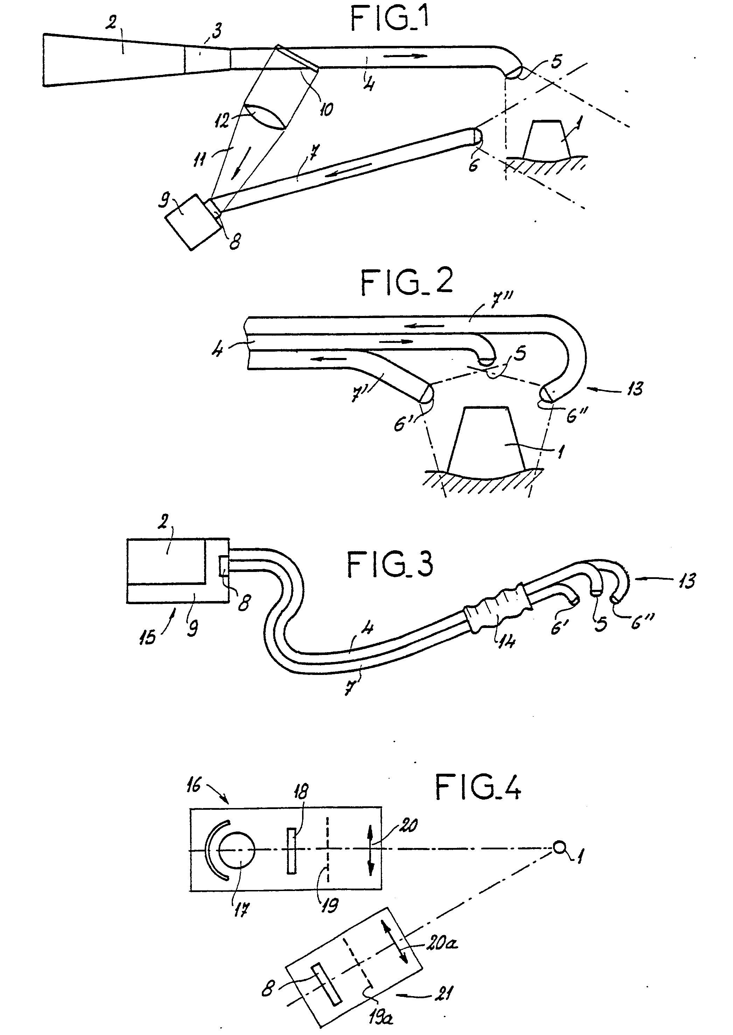

Les figures 1 à 13 sont relatives à diverses formes de réalisation de la partie optique, permettant une analyse en trois dimensions, par interférométrie, des détails de forme et dimension d'un objet qui, dans les premiers exemples, est un moignon de dent taillé (1), destiné à recevoir une couronne.

La figure 1 montre que dans ce but l'on peut utiliser une source (2) de lumière qui après avoir traversé un filtre (3) est dirigé, par une première fibre optique (4), vers la zone du moignon (1) à analyser. Le faisceau d'ondes incident est dispersé sur la zone à analyser par une lentille (5), fixée à l'extrémité de la fibre optique (4). Une autre lentille (6) concentre le faisceau réfléchi, pour le recueillir dans une seconde fibre optique (7) qui le dirige sur un capteur (8), associé à un convertisseur analogique-numérique (9). Un miroir semi-transparent (10), interposé sur le trajet du faisceau incident, dévie une fraction de ce dernier pour obtenir un faisceau de référence (11), qu'une autre lentille (12) concentre sur le capteur (8).

Pour capter le faisceau d'ondes réfléchi, la tête d'analyse (13) c'est-à-dire la partie de l'appareil amenée à l'intérieur de la bouche à proximité de la zone à analyser peut posséder deux lentilles réceptrices (6' et 6"), fixées respectivement aux extrémités de deux fibres optiques (7' et 7") qui guident le faisceau réfléchi, divisé ici en deux fractions (voir figure 2). Deux faces opposées du moignon (1) peuvent être ainsi analysées simultanément. La tête d'analyse (13) est munie d'une poignée de tenue (14) et raccordée par les fibres optiques (4 et 7), à un boîtier (15) situé à l'extérieur de la bouche et renfermant la source émettrice (2) ainsi que le capteur (8) et le convertisseur (9) - voir figure 3.

Chacune des fibres optiques (4 et 7, ou 7' et 7"), doit être gainée pour éviter tous risques oculaires.

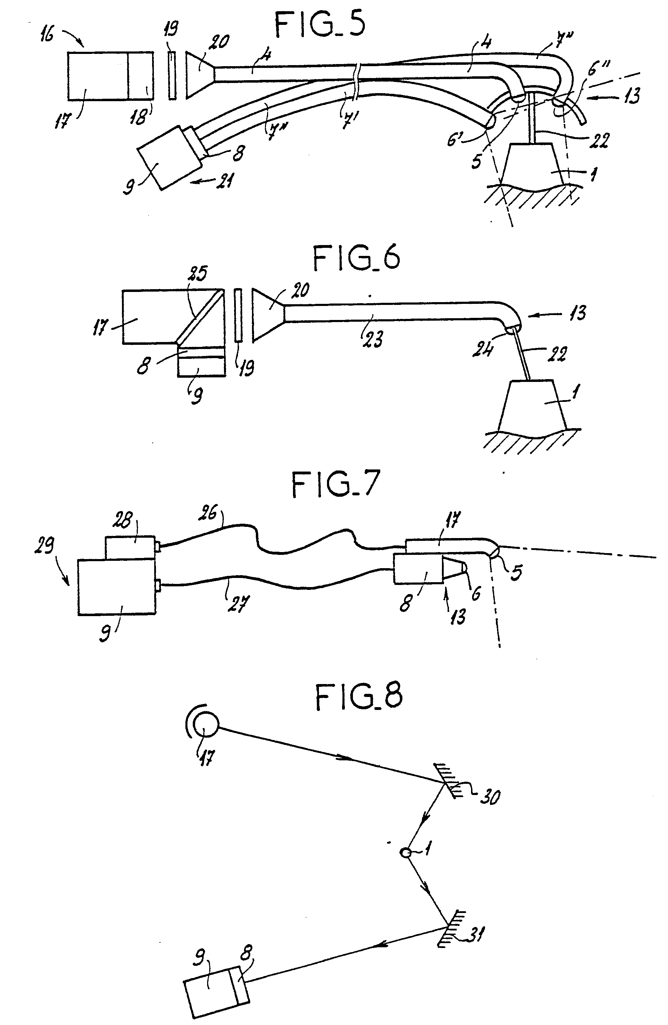

La figure 4 illustre le principe d'un appareil utilisant une source de lumière non cohérente. Les moyens émetteurs d'ondes, désignés dans leur ensemble par le repère (16), comprennent ici une source lumineuse (17) par exemple à halogène, un condensateur de lumière (18), une trame de repérage (19) et un objectif (20). Les moyens récepteurs d'ondes, désignés dans leur ensemble par le repère (21), comprennent un objectif (20a), une trame de repérage (19a) et le capteur (8), toujours associé au convertisseur analogique-numérique. Les deux trames de repérage (19 et 19a), par exemple du type « micro moiré », doivent avoir un pas connu à $10^{-3}$ mm près au minimun. Le nombre de franges ou quadrillage doit être déterminé avec précision (de 1 mm à $10^{-3}$ mm, la valeur idéale étant $10^{-2}$ mm). La distance séparant les deux centres optiques doit être connue à 0,5 mm près, et la focale doit être connue au mm près. Ces différents facteurs sont fixés et définis constructivement. Le « moiré » peut présenter, pour l'analyse des dents, un problème de réflexion, dû à ce que la lumière incidente est de type blanche. Pour éviter ceci, la source (17) utilise de la lumière dans une gamme d'ondes ne correspondant à aucune couleur présente dans la bouche, notamment en associant la source de lumière (17) à un filtre permettant de travailler dans le bleu ou le vert, ou entre le vert et le bleu. Il reste à déterminer ou fixer la distance séparant le plan d'analyse du capteur (8) du plan de référence, à situer toujours en dessous du rebord inférieur de la future couronne, cette dernière distance devant être connue au mm près. Une solution consiste à fixer, sur la tête d'analyse (13), un pointeau (22) de longueur convenablement choisie, destiné à prendre appui sur le moignon de dent (1) à analyser, comme le montre la figure 5 représentant un montage pratique correspondant au principe de la figure 4, et utilisant des fibres optiques (4, 7', 7") ainsi que des lentilles (5, 6', 6") comme dans le cas précédent.

Un autre montage possible est illustré par la figure 6, où une seule fibre optique (23), portant une lentille (24) à son extrémité munie du pointeau (22), guide simultanément le faisceau incident et le faisceau réfléchi, ce dernier étant renvoyé par un miroir semi-transparent (25) sur le capteur (8) associé au convertisseur (9).

Les fibres optiques peuvent être aussi supprimées, comme montré sur la figure 7, en réalisant une tête d'analyse (13) incluant la source lumineuse (17) et le capteur (8), sur lesquels sont directement montées les lentilles respectives (5 et 6). Cette tête (13) est raccordée par des liaisons électriques (26 et 27) à une alimentation électrique (28) et au convertisseur analogique-numérique (9), appartenant à un boîtier extérieur (29).

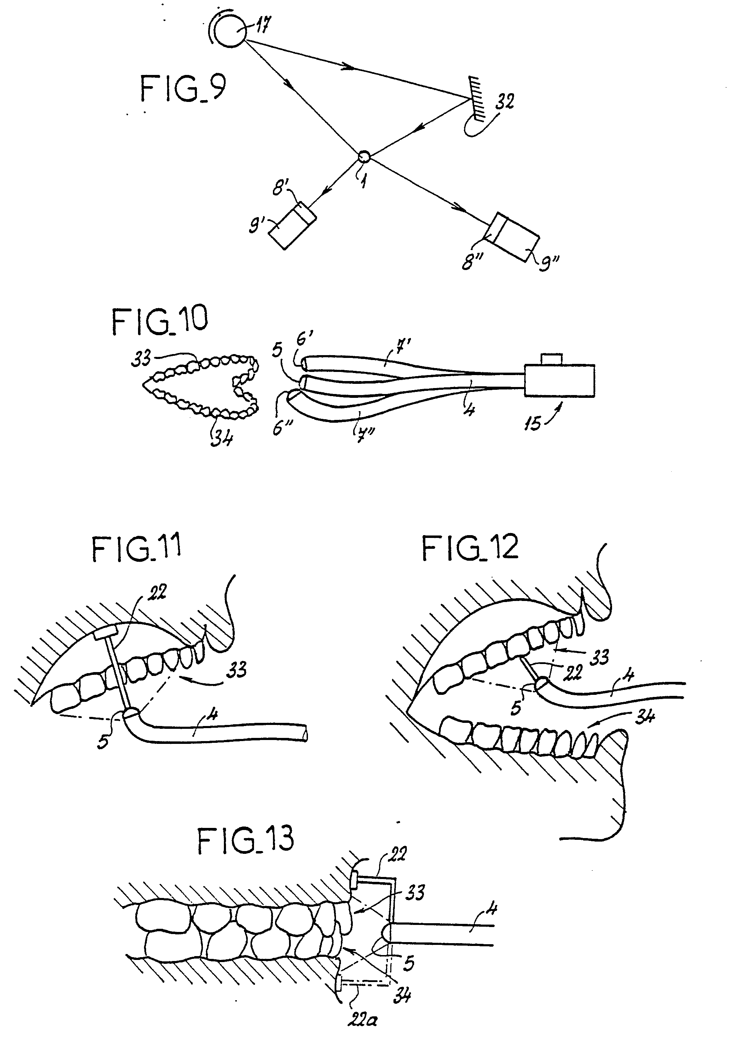

Comme le montre la figure 8, un système de deux miroirs (30 et 31), ajouté au système optique réalisé selon les principes décrits précédemment, pemet de dévier le faisceau incident, et le faisceau réfléchi, de manière à permettre l'analyse de la face de l'objet (1) opposée au côté directement accessible par la source (17) et le capteur (8). En variante, comme illustré par la figure 9, la combinaison d'une source lumineuse (17) d'un unique miroir (32) et de deux capteurs (8' et 8"), associés respectivement à des convertisseurs analogique-numérique (9' et 9"), permet l'analyse simultanée de deux faces opposées de l'objet (1).

Alors que l'objet (1) à analyser était jusqu'ici supposé de faibles dimensions, les figures 10 et suivantes montrent comment le système optique peut être adapté à l'analyse d'une zone plus étendue, comprenant en l'occurrence l'arcade supérieure (33) et/ou l'arcade inférieure (34), pour procéder à une analyse de ces arcades en vue de situer l'ensemble de la prothèse dans le milieu buccal. Les principes de réalisation de l'appareil restent inchangés : un boîtier (15) est relié, par une fibre optique (4), à une lentille (5) diffusant le faisceau incident, tandis que d'autres lentilles (6' et 6") sont fixées au point de départ des fibres optiques (7' et 7") qui guident les faisceaux réfléchis. L'optique doit être plus « large », et le pointeau (22), lorsqu'il est recouru à cette solution, doit être adapté à l'optique et au point d'appui choisi. Ainsi peut être réalisé un appui palatin (voir figure 11) ou un appui dentaire (figure 12), ou encore éventuellement un appui vestibulaire (non représenté). Dans le même but de situer la prothèse dans son environnement, est encore réalisable une analyse de l'occlusion, frontale ou latérale, avec un élément d'appui vestibulaire (22 ou 22a) - voir figure 13, ainsi qu'une analyse de la face, réalisée après avoir placé sur le patient un certain nombre de repères faciaux. Des repères peuvent être notamment placés sur les dents (incisives et prémolaires), à la base du maxillaire inférieur, et au niveau du condyle ou du trou auditif, pour réaliser une analyse dynamique avec mouvements progressifs de la mâchoire (mouvements en occlusion et extrêmes).

Dans la mesure où l'information est prise séparément par plusieurs capteurs, il est nécessaire d'associer les images entre elles. Pour cela on peut réaliser un positionnement par rapport à un élément de référence, tel que le pointeau (22), ou encore par rapport au contour de dent le mieux capté. C'est seulement à la suite d'une telle opération de superposition que l'on peut être certain d'avoir déterminé la totalité de la forme. Pour cela il suffit d'étudier l'« espace-point », c'est-à-dire de vérifier si l'espace entre deux points est constant. Si ce n'est pas le cas, il y insuffisance d'information.

La corrélation sera effectuée en rejoignant les points des images à associer qui sont les plus rapprochés, ceci en respectant la courbure des zones voisines sur les dents ou les gencives, le nombre de points déterminant cette courbure étant fonction de la précision de la méhode.

Une autre modification consiste à augmenter les valeurs en fonction des exigences de l'insertion de la prothèse (petites contre-dépouilles) et des propriétés physiques des ciments de scellement utilisés. Si l'on emploie des métaux « à mémoire », cette dernière opération devient inutile.

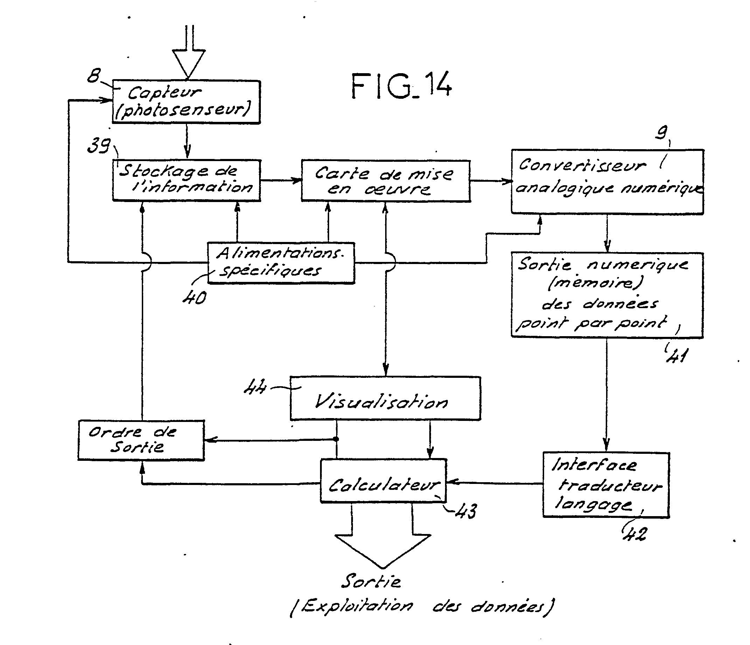

La figure 14 représente plus en détail les moyens de réception des ondes réfléchies et de conversion analogique-numérique, permettant une analyse optique rapide et répétitive, avec visualisation simultanée pour permettre le choix de la meilleure image. Le capteur (8), recevant le faisceau réfléchi par l'objet à analyser, est ici un photosenseur du type CCD matriciel, délivrant une information qui est stockée en (39) avant de parvenir au convertisseur analogique-numérique proprement dit (9), ces différentes parties ayant toutes leurs alimentations spécifiques indiquées en (40). Le convertisseur (9) est raccordé à un circuit de sortie (41), relié par une interface (42) au calculateur (43). De plus, des moyens de visualisation (44), réalisées sous la forme d'un écran moniteur « vidéo », de préférence avec image en couleur, sont reliés de façon interactive aux circuits de stockage (43), pour contrôler le transfert d'informations vers le convertisseur (9).

Le système d'analyse avec photosenseur CCD, dont le principe est donné par la figure 14, nécessite d'avoir au minimum 100 niveaux de gris avec un système « anti-blowing », pour obtenir suffisamment d'information. Les coordonnées « x » et « y » sont données automatiquement dans le plan. Elles sont corrigées ultérieurement en fonction de la focale d'analyse utilisée, et de l'éloignement du point considéré par rapport à l'axe optique. La coordonnée « z » est le résultat d'une transformée du type Fourier. Les algorithmes mathématiques à utiliser ici sont actuellement connus.

Tous les moyens décrits jusqu'à présent permettent l'obtention d'une empreinte optique en trois dimensions notamment d'un moignon de dent (1), et sa traduction sous une forme numérique exploitable par des moyens de traitement automatique de l'information. Ce traitement numérique doit, en considérant l'application à la réalisation automatique d'une couronne, prendre encore en considération d'autres facteurs, théoriques et réels.

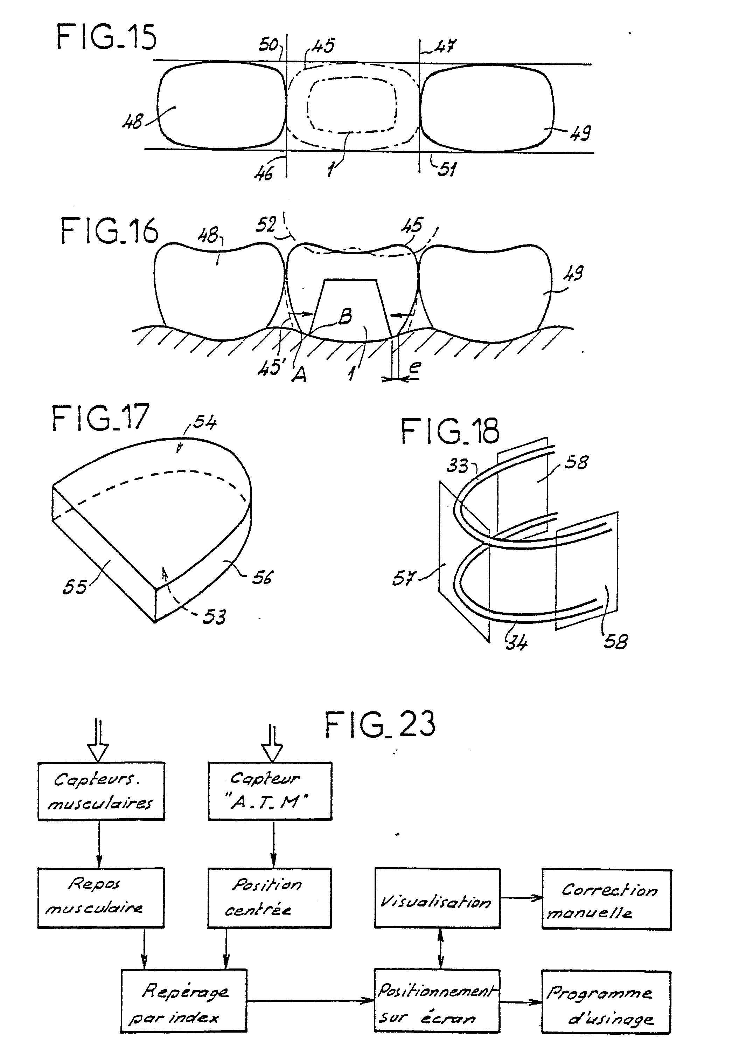

En particulier, comme le montre la figure 15, doit être déterminée l'enveloppe de la future couronne (45), c'est-à-dire le volume à l'intérieur duquel celle-ci doit s'inscrire. Ce volume est défini par des plans, entre autres un plan antérieur (46) et un plan postérieur (47), théoriquement tangents aux dents adjacentes (48 et 49), aux points de contact avec la dent à reconstituer, et dont la détermination pratique peut prendre en compte des facteurs correctifs, dus aux mouvements physiologiques et au diastème. Le volume en question est aussi délimité par un plan latéral palatin ou lingual (50), et par un plan latéral vestibulaire (51), tangents, l'un et l'autre aux dents antérieure (48) et postérieure (49) ; ici également peuvent intervenir des facteurs correctifs, liés au type de dent à reconstituer, ces facteurs pouvant être dégressifs par exemple dans le cas d'une incisive latérale supérieure. Pour définir complètement l'enveloppe, sensiblement parallélépipédique, dans laquelle s'inscrit la couronne théorique, il reste encore à définir un plan inférieur, donné par la limite singulaire de la couronne, tracé automatiquement ou manuellement sur moniteur, et un plan supérieur ou occlusal, déterminé en occlusion et défini comme le plan passant par le point le plus élevé de la surface occlusale de la dent antagoniste ou, en cas d'absence de cette dernière, par la limite supérieure des dents antérieure et postérieure.

Pour affiner la détermination du plan supérieur, on propose ici une méthode de détermination de la cinématique mandibulaire :

En faisant une empreinte de la mandibule avec précision, il est possible de déterminer les facettes d'usure de chaque dent. Ces facettes correspondent aux plans de glissement de la mandibule sur le maxilllaire supérieur. Ces facettes sont donc l'empreinte des mouvements mandibulaires. En les étudiant dans leur ensemble il est connu depuis longtemps que l'on peut reconstituer les mouvements avec une totale exactitude.

Pour repérer ces zones il suffit de faire serrer les deux maxillaires. Les points de contact se trouvent forcément sur ces facettes. Si ces plans se retrouvent sur chaque dent et correspondent entre eux, ce sont forcément les plans d'usure de la mandibule. Connaissant mathématiquement les interrelations entre surface d'usure et mouvements mandibulaires de ces facettes, on connaîtra les mouvements sans être obligé de faire une étude dynamique des mouvements des repères les uns par rapport aux autres.

De la même manière il découlera automatiquement le plan supérieur de l'enveloppe. En appliquant à ce plan les règles connues de l'occlusion, ne seront retenus comme zone de contact dent antagoniste-couronne que les points du type tripodisme, « long centric » ou autre stabilisant l'arcade, permettant la mastification et permettant des déplacements normaux de la mandibule.

L'on obtient ainsi six plans, auxquels doivent être tangentes les diverses faces extérieures d'une couronne théorique, pouvant être désormais entièrement déterminée en partant d'une forme de dent théorique mise en mémoire, ou en partant de l'analyse de la dent symétrique existante après correction « gauche-droite ». Au cas éventuel où la dent était intacte avant l'opération de taille, il suffirait de réaliser une empreinte optique avant la taille et une empreinte optique après la taille. Pour un ensemble correspondant à plusieurs dents, il suffit de diviser l'espace considéré en une pluralité d'enveloppes unitaires.

Une fois déterminée la forme extérieure de la couronne théorique, il convient de l'adapter, comme l'illustre la figure 16, à la forme et à la position du moignon de dent (1). L'opération consiste à faire coïncider le bord inférieur (A) de la couronne (45), situé dans le plan inférieur de l'enveloppe, avec le contour (B) du moignon (1), en respectant une épaisseur minimale de matière (e) au point le plus bas. Pour cela, le profil extérieur théorique de la couronne (45') subit une correction progressive, lui conservant une allure de courbe régulière tout en lui permettant de joindre la zone de contact avec les dents adjacentes (48 et 49), d'une part, et la limite inférieure désirée, d'autre part.

En ce qui concerne la limite supérieure, une première correction à effectuer consiste à centrer la gouttière de la dent théorique sur la ligne générale des gouttières des dents de l'arcade. Une deuxième correction est celle d'occlusion statique ; elle représente une adaptation aux surfaces occlusales des dents antagonistes telle que (52) - figure 16, saisie par des moyens décrits plus loin. Une troisième correction consiste à faire un lissage mathématique en incluant la surface obtenue à l'ensemble des arcades en occlusion et en induisant des mouvements latéraux et antéro-postérieurs effectuant un lissage des données. Il est à noter que l'enveloppe peut être déterminée aussi par l'adjonction d'une coiffe déformable sur le moignon (1), le plan supérieur ou occlusal étant dans ce cas analysé en trois dimensions après avoir demandé au patient d'effectuer tous les mouvements nécessaires.

Dans le cas d'une prothèse mobile, comme le montre la figure 17, la définition de l'enveloppe est partiellement différente : on distingue toujours un plan inférieur (53), un plan supérieur (54) et un plan postérieur (55), mais les plans latéraux et antérieur sont réunis en une seule surface enveloppe incurvée (56), qui sera déterminée comme étant sensiblement tangente à la crête gingivale, et toutefois espacée de celle-ci de quelques millimètres, cet espacement étant fonction de la partie la plus vestibulée de la prothèse (bord libre des incisives ou rebord de la résine, etc.). La limite inférieure de la surface (56) devra être fixée à une profondeur non traumatisante, fonction de l'empreinte optique définissant le plan inférieur (53). Au moment de la prise de cette empreinte, il sera souhaitable de faire jouer les muscles buccaux pour que son intersection avec la surface (56) ne soit pas traumatisante. La plan postérieur (55) sera déterminé par les éléments anatomiques situant la limite théorique d'une prothèse (tubérosité rétro-molaire, trigone, etc.). Enfin, le plan supérieur (54), c'est-à-dire le plan d'occlusion réelle, décrite ci-après en référence aux figures 18 et suivantes.

Dans ce but, s'il existe encore des contacts dentaires, l'on réalise des empreintes optiques des arcades supérieure (33) et inférieure (34), comme déjà décrit plus haut, ainsi que des empreintes optiques antérieures et latérales définissant des « claies » antérieure (57) et latérales (58). Ensuite l'on procède à l'ajustement des empreintes supérieure et inférieure l'une par rapport à l'autre, les claies permettant le positionnement mathématique exact d'un maxillaire par rapport à l'autre, d'où obtention de l'occlusion « statique ».

S'il n'existe pas d'antagoniste, dans le cas d'une prothèse fixe, on se reportera au cas traité précédemment, alors que pour une prothèse mobile (complète) on procédera comme décrit plus haut, en déclenchant la prise d'empreinte de la claie de préférence au pied pour bien fixer les rapports inter-maxillaires au bon moment.

Pour la détermination de l'occlusion « dynamique », les mouvements des mâchoires peuvent être enregistrés selon un mode opératoire sensiblement identique, par analyse faciale du patient sur lequel on aura placé un certain nombre de repères buccaux. Si cette analyse faciale se fait en rayons X, il suffit d'avoir trois repères radio-opaques, liés aux maxillaires et visibles à la prise d'empreinte optique. S'il s'agit de repères émetteurs, il faut que la table d'analyse soit sensible à la longueur d'onde d'émission, et que ces repères soient visibles à la prise d'empreinte optique. S'il s'agit de repères classiques, il faut qu'ils soient visibles à tout moment durant l'analyse des mouvements. Dans chaque cas, les repères doivent être fixes à quelque 100 μm près, entre le début et la fin de l'analyse. Si ces repères ne sont que intra-buccaux, il faut qu'ils soient visibles à tout instant durant l'analyse des mouvements.

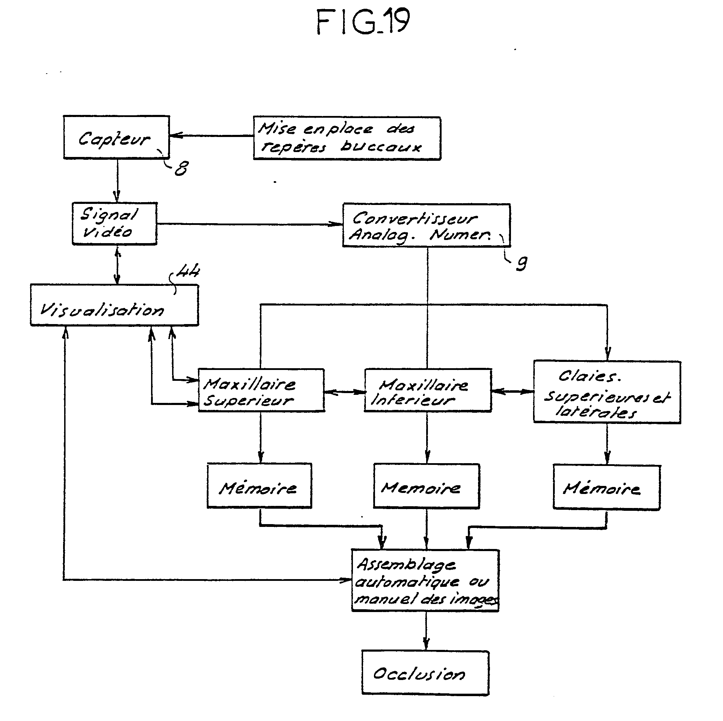

Le schéma de la figure 19 illustre l'exécution de cette dernière opération, effectuée en réalisant un contrôle par les moyens de visualisation (44).

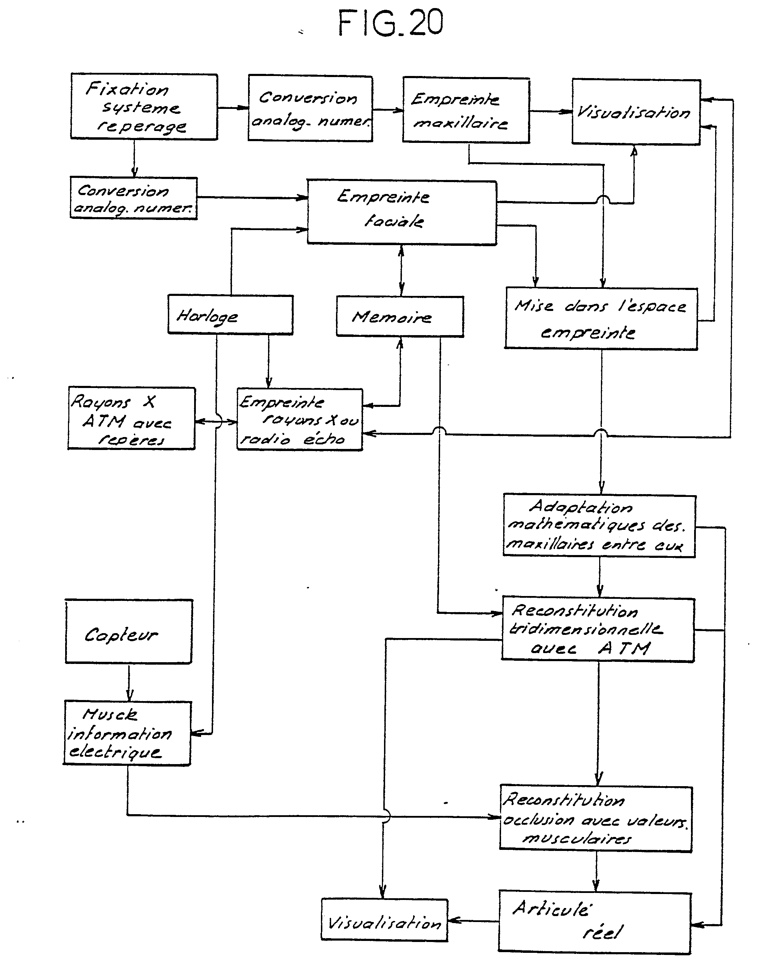

Dans tous les cas sains, cette analyse évite une étude directe de l'articulation temporaux-maxilaire. Dans les cas pathologiques, il sera cependant judicieux d'adjoindre des moyens d'étude musculaire (tension, etc.) et d'étude de cette articulation, le processus à suivre étant illustré par le schéma de la figure 20. Dans ce dernier cas, on utilisera pour les repères internes et externes un système de fixation endo-buccal sur dent ou sur gencive, collant ou à ventouse, et après enregistrement des mouvements faciaux des points externes, on prendra deux empreintes optiques, l'une avec les points de repère, l'autre pour la prothèse elle-même. Il suffit alors de positionner mathématiquement les empreintes l'une par rapport à l'autre, en ne retenant que les repères. Les mouvements faciaux permettront de donner les mouvements des mandibules dans l'espace. L'étude de l'articulation temporaux-maxillaire permettra de suivre le cas pathologique par rapport à l'occlusion, et de chercher une meilleure occlusion. La même remarque peut être faite pour l'étude musculaire.

En variante, on peut aussi appliquer ici la méthode des facettes d'usure expliquée précédemment, et retrouver ainsi mathématiquement tous les mouvements de la mandibule sans avoir à les étudier de façon dynamique.

En ce qui concerne la détermination des couleurs, la teinte d'une dent sera évaluée par un procédé connu de thermographie, en utilisant en particulier un récepteur à infrarouge à grande ouverture ou en suscitant un éclairement infrarouge. Les couleurs déterminées automatiquement seront adressées, après visualisation (en couleur réelle), à la mémoire puis au stock de dents. Cette même détermination peut induire la fabrication automatique de surfaces esthétiques (inclusions automatiques de couches successives de résine ou céramique, suivie de cuissons automatiques de dents).

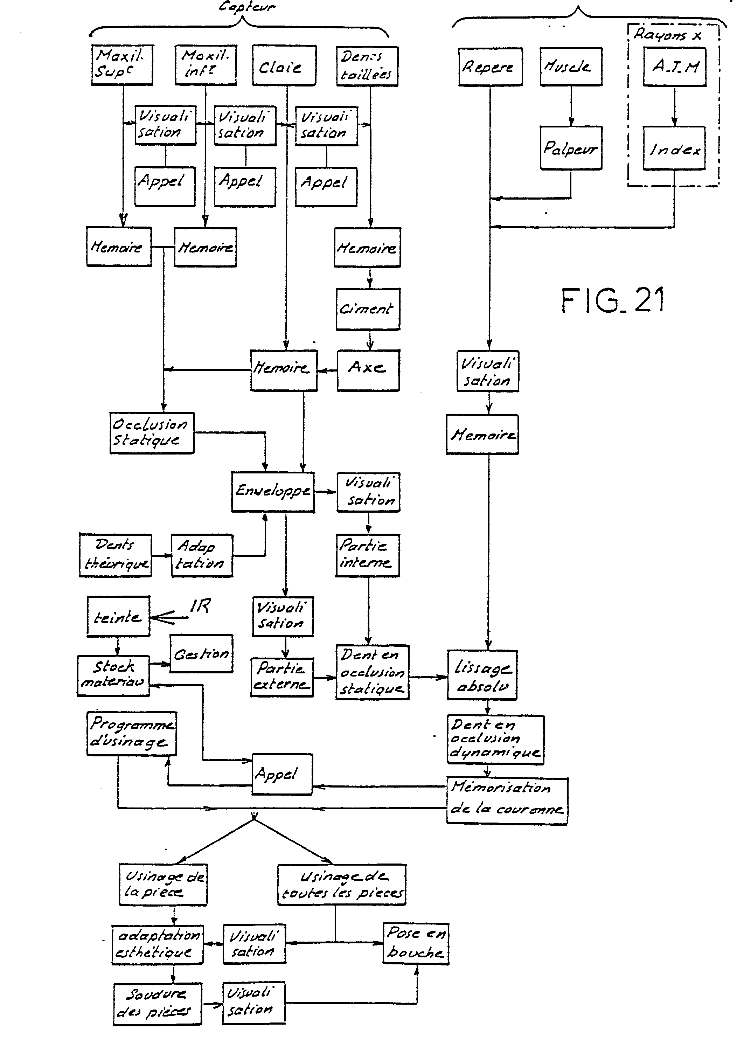

Le schéma de la figure 21 résume l'ensemble des opérations relatives à la définition des caractéristiques, d'une prothèse fixe, à sa réalisation et à son adaptation, à partir de prises d'empreintes optiques et autres données obtenues par les moyens précédents. Des exemples concrets de fabrication de prothèses fixes, notamment couronnes, seront décrits plus loin.

On s'intéressera, auparavant, aux principes de fabrication de prothèses mobiles, en considérant tout d'abord une prothèse mobile avec antagoniste.

Dans ce cas, les informations devant être reçues sont : l'empreinte optique des deux maxillaires, les claies frontales et latérales, les mouvements généraux des maxillaires. On effectue les opérations comme suit :

— On prend l'empreinte optique des deux maxillaires et des claies. Après les avoir visualisées et choisi la meilleure image, on effectue la conversion analogique-numérique, et l'on stocke les résultats. Il est nécessaire de bien faire jouer les muscles pour que la limite inférieure de la surface enveloppe incurvée (56) soit exacte.

— On fait une détermination d'occlusion statique et dynamique pour limiter encore plus le volume enveloppe.

— Un appel à la mémoire théorique ou le tracé manuel sur moniteur permettra de proposer un tracé de prothèse sur ce type de maxillaire et dans l'enveloppe considérée.

— Un appel à la mémoire théorique permettra le choix des dents selon le même principe que pour une prothèse fixe (adaptation à l'occlusion, aux mouvements mandibulaires, à la teinte).

— Pour des raisons esthétiques, l'usinage sera séparé pour les dents d'une part et la plaque d'autre part.

— On adoptera un système de fixation usiné sur la plaque et dont les contreparties seront usinées sur les dents. Le type de fixation choisi doit être stable et démontable. La fixation sera automatique ou manuelle.

— Une étude échographique peut permettre de connaître les zones de décharge (épaisseur gingivale).

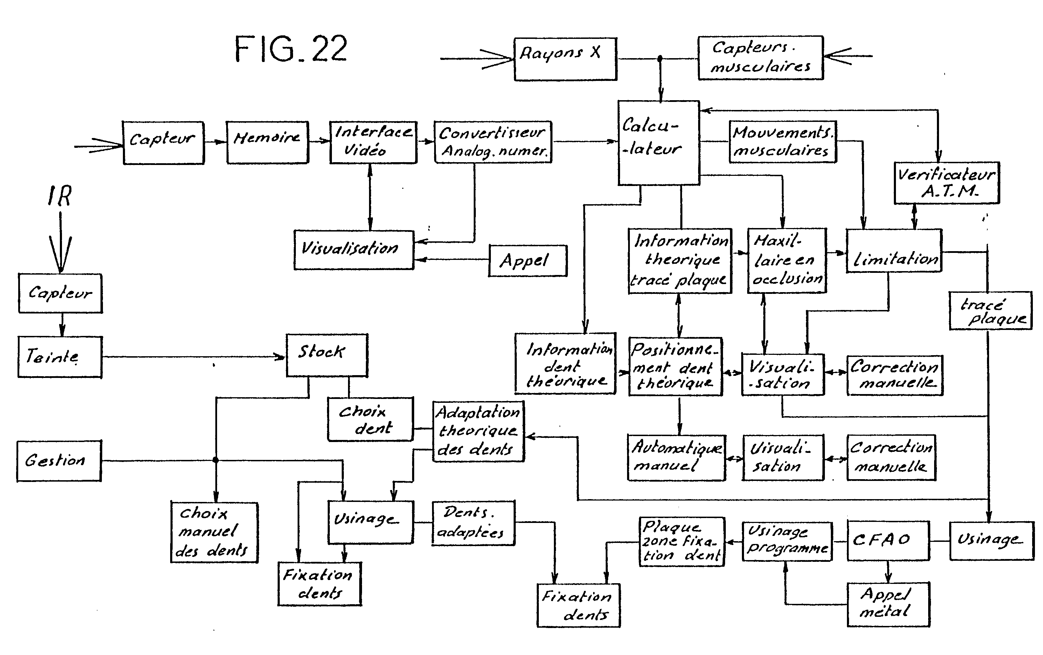

Chacune de ces étapes sera contrôlée à tout moment et corrigée éventuellement sur l'écran moniteur de visualisation, comme le montre le schéma de la figure 22 qui résume l'ensemble des opérations. Le système de repérage d'usinage sera précisé plus loin.

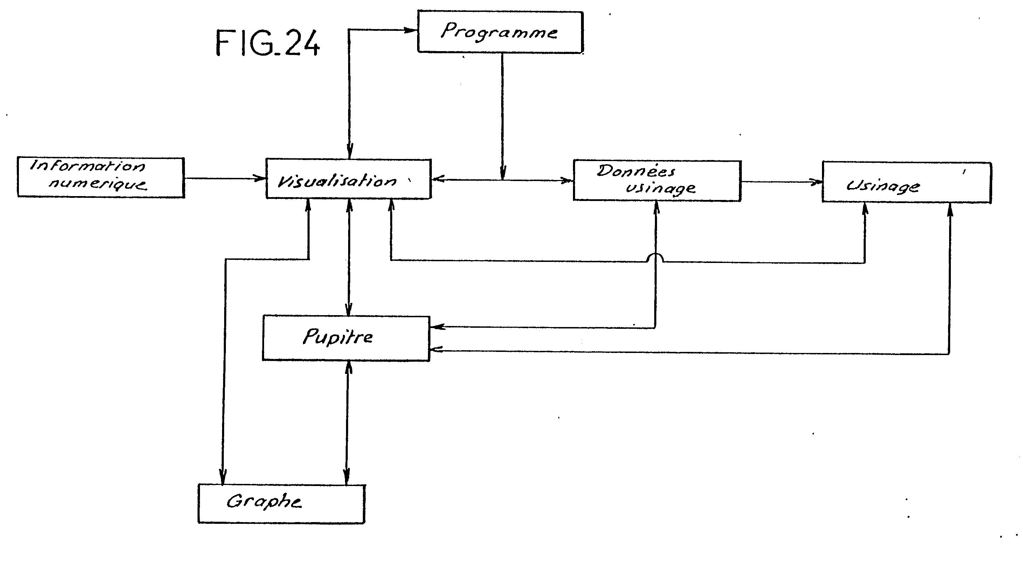

Pour la fabrication d'une prothèse mobile sans antagoniste, les opérations sont sensiblement identiques mais nécessitent une évaluation plus exacte de la position de repos musculaire de la bouche, puis de celle de l'occlusion. Pour cette raison, il est ici proposé un système identique au précédent, à l'exception de foncitons complémentaires illustrées par le schéma de la figure 23, pour opérer une analyse plus stricte au niveau de l'occlusion ;

— Analyse de la position de repos avec l'aide de capteurs musculaires et d'une analyse de l'articulation temporaux-maxillaire.

— Réduction de 2 mm par exemple de la position obtenue, donnant la position d'occlusion.

— Réunion théorique des deux maxillaires sur cette dernière position.

S'il reste des dents servant de support pour la prothèse à fabriquer, le calculateur déterminera l'axe d'insertion idéal, offrant le maximun de stabilité, comme résultante de l'ensemble des axes dentaires pris séparément. Dans la mesure où l'insertion est impossible, il y aura positionnement d'attachement sur les dents les plus responsables. Enfin le calculateur choisira, pour le tracé des crochets ou le point d'ancrage des attachements, la meilleure situation.

En orthopédie dento-faciale, l'empreinte optique permet de traiter réellement l'étude des moulages de façon automatique. Elle permet d'aboutir à un diagnostic, et à la proposition d'un traitement dent par dent en associant les données radiographiques, échographiques et thermographiques. En faisant l'empreinte optique de l'arcade et de maxillaires, on pourra faire un calcul automatique des constantes servant au diagnostic. En faisant une empreinte de la face, de façon automatique on en déterminera les constantes faciales fondamentales. En associant l'empreinte optique buccale et faciale à l'analyse musculaire et radiographique, il en découlera une automatisation du diagnostic puis du traitement, chose qui était impossible jusqu'à présent. Compte tenu de l'empreinte optique des maxillaires, et des empreintes radiologiques des dents prises séparément, il y aura usinage automatique des crochets de traction des dents à déplacer et de leurs points de fixation sur les dents ou sur une prothèse mobile. En faisant une empreinte optique chaque année à un même enfant, le diagnostic se fera automatiquement l'année où l'intervention deviendra nécessaire. Enfin, en prévoyant les résultats de façon empirique, il sera possible de contrôler chaque étape du traitement.

Dans le domaine de la dentisterie opératoire, plutôt que d'utiliser la photographie (selon le procédé du brevet US-A-3 861 044 signalé dans l'introduction), le dispositif selon la présente invention permet de réaliser une empreinte optique, donnant directement une image en trois dimensions, sans utiliser le support intermédiaire qu'est la photographie et en stockant au contraire directement l'information, moyennant seulement une conversion analogique-numérique. Pour déterminer le volume utile de l'obturation à réaliser, on procédera comme pour une prothèse, mais d'une façon plus rapide :
— 1er temps : détermination du volume.
— 2e temps : détermination des plans enveloppes (cavités composées).
— 3e temps : détermination de l'occlusion statique et dynamique.
— 4e temps : usinage de l'insert (ou « inlay ») d'obturation.

Le mode opératoire est donc identique à celui de la réalisation d'une prothèse fixe. Le volume de la chambre pulpaire sera évalué de la même façon afin de calibrer les instruments.

En paradontologie, les moyens d'investigation proposés par la présente invention permettent de résoudre le diagnostic « articulation temporaux-maxillaire/muscle/articulé dentaire », mais aussi celui de la dent par rapport à son support osseux. Dans le premier cas, après avoir fait un repérage de l'articulé occlusal, on y associe la position du condyle dans la cavité glénoïde et l'état de tension musculaire. On saura ainsi s'il existe une dissymétrie articulaire ou musculaire ou toute autre pathologie liée à cet ensemble. Dans le cas du diagnostic dentaire, on déterminera par échographie ou par xérographie le contour osseux par rapport au contour dentaire et muqueux, et l'on mesurera la mobilité dentaire par empreintes optiques successives.

Le système de programmation et de communication avec l'opérateur doit évidemment être adapté à la nature des informations traitées (formes définies numériquement), en permettant la visualisation, le contrôle et la sauvegarde du travail, essentiellement en utilisant un pupitre avec clavier alphanumérique et de commande de fonctions, ainsi qu'un écran de visualisation et des moyens graphiques interactifs, permettant de « manipuler » l'image : translation, changement d'échelle, rotation... Les données seront traitées rapidement, en « temps réel », tout en permettant de stocker le travail effectué, pour la constitution du fichier « clients » et l'exécution d'autres tâches de gestion du cabinet dentaire ou médical. Les images à traiter et les formes à usiner se ramènent essentiellement à des surfaces et volumes. Les surfaces complexes seront définies comme une fraction d'interpolation d'un réseau de points et de courbes, sur lesquels des contraintes transversales de tangence et de courbure seront imposées. Les volumes complexes devront délivrer des volumes polyédriques imposés par les limites de l'enveloppe optique et par les possibilités d'insertion de la pièce prothétique. Une dernière fonction moins abstraite sera la préparation de l'usinage par commande numérique, à partir des éléments stockés dans la mémoire, avec contrôle permanent, l'opérateur n'ayant pas à choisir le travail car il sera présélectionné (fonctions réduites).

Le schéma-bloc de la figure 24 résume à titre d'information le principe d'usinage automatique d'une prothèse et de contrôle d'usinage.

Plus particulièrement, deux types d'usinage sont à envisager, dans le domaine de la prothèse et de la dentisterie opératoire, à savoir l'usinage du support et l'usinage de la ou des pièces esthétiques. L'usinage du support doit s'adapter aux surfaces existantes, telles que celles du moignon, déterminées par les moyens exposés précédemment. La partie en contact avec la bouche suivra les règles connues. Pour la partie supérieure, plusieurs possibilités sont offertes :

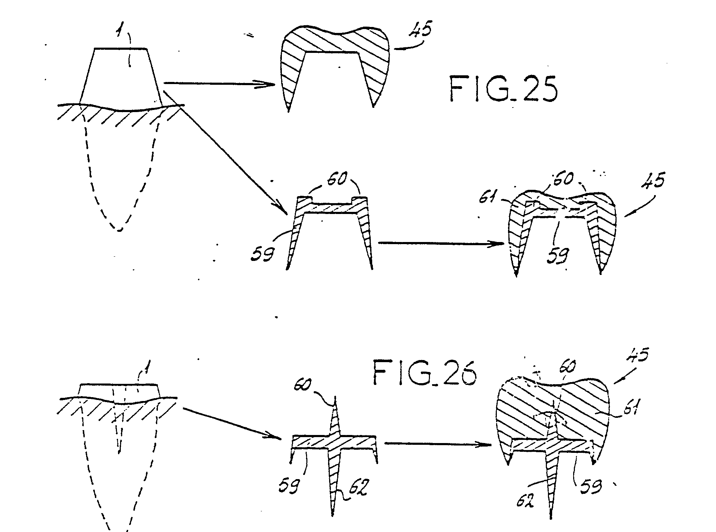

Comme montré en haut de la figure 25, si le matériau utilisé est esthétiquement et biologiquement acceptable, le support est usiné en fonction de toutes les données à prendre en considération, pour constituer lui-même la totalité de la couronne (45) à réaliser, s'adaptant sur le moignon (1).

Le support (59) peut aussi, comme montré au bas de la figure 25, comporter des points de fixation (60), usinés pour la mise en place d'une pièce esthétique (61) qui viendra recouvrir exactement le support (59), l'ensemble constituant la couronne (45).

Dans le cas d'un moignon (1) trop réduit, le support (59), peut encore comporter un pivot de fixation (62), éventuellement fileté, comme le montre la figure 26.

Comme il va de soi, l'invention ne se limite pas aux seules formes d'exécution de ce dispositif de prise d'empreinte qui ont été décrites ci-dessus, à titre d'exemples ; elle en embrasse, au contraire, toutes les variantes de réalisation et d'application fondées sur les caractéristiques essentielles énoncées dans les revendications ci-jointes.

En particulier :

— La fonction du capeur (8) peut être effectuée en temps différé par la corrélation vectorielle entre deux images radiographiques ou photographiques, prises sous des angles différents.

— Le convertisseur analogique-numérique (9), réalisé sous la forme d'un microprocesseur, peut être incorporé à l'ensemble endobuccal, dans un dispositif similaire à celui de la figure 7.

— Il est possible d'utiliser une première fibre optique de section circulaire pour guider le faisceau incident, et une seconde fibre optique de section annulaire, entourant la première, pour guider le faisceau réfléchi, au lieu de fibres optiques juxtaposées.

— Dans le système optique, il est possible d'utiliser non seulement des miroirs, mais aussi des prismes en association avec les fibres optiques, pour permettre une observation latérale dans des parties de la bouche d'accessibilité difficile.

## Revendications

1. Dispositif de prise d'empreinte par des moyens optiques, notamment en vue de la réalisation automatique de prothèses, et encore plus particulièrement la réalisation de prothèses telles que les couronnes utilisées en art dentaire, ce dispositif comprenant en combinaison, des moyens émetteurs d'ondes lumineuses non traumatisantes (17) ; une tête d'analyse (13) incluant des moyens transmetteurs (4, 5) pour diriger lesdites ondes vers la partie du corps, telle qu'emplacement de dent (1), à analyser, et des moyens récepteurs (6, 7) des ondes lumineuses réfléchies par cette partie du corps, dirigeant lesdites ondes sur un capteur (8), ledit capteur étant associé à un convertisseur analogique-numérique (9), permettant d'obtenir sous forme de signaux numériques une représentation de la forme, dans les trois dimensions de l'espace, de la partie du corps à analyser, et des moyens d'analyse et de traitement (39 à 44) des signaux numériques obtenus, notamment en vue de la commande automatique d'une machine-outil à commande numérique (70) pour l'usinage d'une prothèse, telle qu'une prothèse dentaire (45) adaptée exactement à la partie du corps analysée (1), caractérisé en ce que lesdits moyens émetteurs (17) comprennent une source émettrice de lumière non cohérente et en ce que ledit dispositif comprend en outre, pour permettre l'analyse par interférométrie holographique, des trames de repérage (19, 19a) (connues en soi) associées, respectivement à la source émettrice (17) et au capteur (8), et des moyens de détermination de distance permettant de déterminer ou de fixer la distance entre les moyens optiques de la tête d'analyse (13) et un plan de référence lié à la partie du corps à analyser (1).

2. Dispositif selon la revendication 1, caractérisé en ce que les moyens émetteurs (17), utilisent de la lumière dans une gamme d'ondes ne correspondant à aucune couleur présente dans la bouche, notamment en associant la source de lumière (17) à un filtre permettant par exemple de travailler dans le bleu ou le vert, ou entre le bleu et le vert.

3. Dispositif selon la revendication 1 ou 2, caractérisé en ce que les moyens de détermination de distance associés à la tête d'analyse (23), sont constitués par un émetteur-récepteur d'ultra-sons ou d'infra-rouge, déterminant la distance de ladite tête d'analyse (17) par rapport au plan de référence, au moment précis où est réalisé l'éclairage par la source (17).

4. Dispositif selon la revendication 1 ou 2, caractérisé en ce que les moyens de détermination de distance associés à la tête d'analyse (13), sont constitués par un repère de longueur connue (22), destiné à reposer sur un point de la partie du corps à analyser (1).

5. Dispositif selon l'une quelconque des revendications 1 à 4, caractérisé en ce que la source (17), le capteur (8) et le convertisseur analogique-numérique (9) sont placés dans un boîtier extérieur (15), relié par des fibres optiques (4, 7) à la tête d'analyse (13), telle qu'une tête apte à être amenée dans la bouche d'un patient.

6. Dispositif selon la revendication 5, caractérisé en ce que la tête d'analyse (13) possède deux lentilles réceptrices (6', 6") fixées respectivement aux extrémités de deux fibres (7', 7") qui guident le faisceau réfléchi, divisé en deux fractions, pour analyser simultanément deux faces opposées de la partie du corps (1).

7. Dispositif selon l'une quelconque des revendications 1 à 4, caractérisé en ce qu'il comporte une fibre optique unique (23), portant à son extrémité une lentille 24 qui guide à la fois le faisceau incident et le faisceau réfléchi, ce dernier étant renvoyé par un miroir semi-transparent (25) sur le capteur (8) associé au convertisseur (9).

8. Dispositif selon l'une quelconque des revendications 1 à 3, caractérisé en ce que la tête d'analyse (13) inclut la source d'éclairage (17) et le capteur (8), avec les lentilles respectives (5, 6) montées directement, cette tête (13) étant raccordée par des liaisons électriques (26, 27) avec un boîtier extérieur (29) incluant l'alimentation électrique (28) et le convertisseur analogique-numérique (9).

9. Dispositif selon l'une quelconque des revendications 1 à 8, caractérisé en ce que le système optique est complété par un ou des miroirs (30, 31, 32) pour l'analyse simultanée de deux faces opposées de la partie du corps (1).

10. Dispositif selon l'une quelconque des revendications 1 à 9, caractérisé en ce que le capteur (8) atteint par le faisceau réfléchi, et aussi éventuellement par un faisceau de référence (11), est du type photosenseur à transfert de charge, et plus particulièrement un photosenseur du type CCD matriciel.

11. Dispositif selon la revendication 10, caractérisé en ce que des moyens de stockage de l'information (39) sont insérés entre le capteur (8) et le convertisseur analogique-numérique proprement dit (9), lequel délivre vers les moyens de traitement (43) des signaux de sortie « point par point » (41), transformés par une interface (42) pour leur adaptation aux moyens de traitement numérique (43), des moyens de visualisation (44) des données saisies étant en outre prévus, en liaison avec les moyens de stockage (39) précités.

12. Dispositif selon l'une quelconque des revendications 1 à 11, caractérisé en ce que les moyens d'analyse et de traitement comprennent encore des moyens de détermination et de prise en compte de l'enveloppe ou volume (46, 47, 50, 51, 53 à 55) à l'intérieur duquel doit s'inscrire la prothèse (45) à usiner.

13. Dispositif selon la revendication 12, caractérisé en ce que l'enveloppe ou volume à l'intérieur duquel doit s'inscrire la prothèse (45) à usiner est déterminé latéralement par :

a) les zones de contact (46, 47) des dents voisines (48, 49) et,

b) les plans (50, 51) tangents à l'arcade ou déterminés sur le côté symétrique pour les faces vestibulaires ou linguales ; inférieurement par :

c) les limites inférieures du moignon (1) analysé en bouche, pour le plan inférieur et supérieurement par :

d) un plan supérieur en fonction d'une analyse mathématique issue soit de l'étude des zones d'usure de l'ensemble du maxillaire, soit du déplacement réel de la mandibule.

14. Dispositif selon la revendication 12, caractérisé en ce que les moyens d'analyse et traitement des signaux (43) comprennent encore des moyens de correction automatique de la forme extérieure théorique de la prothèse (45') inscrite dans l'enveloppe précitée, en fonction de données résultant de l'analyse optique, telles que la forme du moignon de dent (1) dans le cas de réalisation d'une couronne.

15. Dispositif selon l'une quelconque des revendications 1 à 14, caractérisé en ce que les moyens d'analyse et de traitement comprennent encore des moyens de détermination de l'occlusion, statique et dynamique.

16. Dispositif selon la revendication 15, caractérisé en ce que l'occlusion est déterminée par repérage des maxillaires supérieur (33) et inférieur (34), prises d'empreinte optique séparées des deux maxillaires, réalisation d'une claie optique antérieure (57) des repères des deux maxillaires, et réunion des deux maxillaires grâce à leurs repérages au niveau de la claie.

17. Dispositif selon l'une quelconque des revendications 1 à 16, caractérisé en ce que les moyens d'analyse et de traitement comprennent encore des moyens de détermination et de prise en compte des couleurs ou teintes de dents.

**Claims**

1. An apparatus for taking impressions by optical means, particularly for the purpose of automatic production of prostheses and even more particularly for the production of prostheses such as the crowns used in dentistry, this apparatus comprising in combination : means to emit non-traumatic light waves (17), an analysing head (13) including transmission means (4, 5) to direct the said light waves towards the part of the body, such as the site of the tooth (1) to be analysed, and reception means (6, 7) for the light waves reflected by this part of the body directing the said waves on to a sensor (8), the said sensor being associated with an analogue-numerical convertor (9) enabling a representation of the three-dimensional shape of the part of the body to be analysed to be obtained in numerical form, and means of analysis and treatment (39 to 44) of the numerical signals obtained, notably for the purpose of automatic control of a numerically-controlled machine-tool (70) to machine a prosthesis such as a dental prosthesis (45) precisely adapted to the part of the body analysed (1), characterized in that the said emitting means (17) comprise an emission source of non-coherent light and in that the said apparatus additionally comprises, to permit analysis by holographic interferometry, location screens (19, 19a) (in themselves known) associated with the emitting source (17) and the sensor (8) respectively, and means of determination of distance enabling determination or fixation of the distance between the optical means of the analysis head (13) and a reference plane associated with the part of the body to be analysed (1).

2. An apparatus according to Claim 1, characterized in that the emitting means (17) employ a wave spectrum that does not correspond to any colour present in the mouth, notably by associating the light source (17) with a filter enabling operation in the blue or the green range for example, or between the blue and the green.

3. An apparatus according to Claim 1 or 2, characterized in that the means of determination of distance associated with the analysis head (13) are formed by an ultrasonic or infra-red emitter-receiver, determining the distance of the said analysis head (13) from the reference plane at the exact moment that illumination by the source (17) takes place.

4. An apparatus according to Claim 1 or 2, characterized in that the means of determination of distance associated with the analysis head (13) are formed by a marker of known length (22), intended to rest on a point of the portion of the body to be analysed (1).

5. An apparatus according to any Claims 1 to 4, characterized in that the source (17), the sensor (8) and the analogue-numerical converter (9) are placed in an external case (15), connected by optical fibers (4, 7) to the analysis head (13), such as a head suitable for placing in the mouth of a patient.

6. An apparatus according to Claim 5, characterized in that the analysis head (13) possesses two receiving lenses (6', 6") fixed to the ends of two optical fibres (7', 7") respectively which fibres guide the reflected beam, divided into two fractions, for simultaneous analysis of two opposite faces of the portion of body (1).

7. An apparatus according to any of Claims 1 to 4, characterized in that it comprises a single optical fibre (23) carrying a lens (24) at its end, which at one and the same time guides the incident beam and the reflected beam, this latter being diverted by a semi-transparent mirror (25) on the sensor (8) associated with the converter (9).

8. An apparatus according to any of Claims 1 to 3, characterized in that the analysis head (13) includes the source of illumination (17) and the sensor (8), with their respective lenses (5, 6) directly mounted, this head (13) being connected by electrical conductors (26, 27) to an external case (29) that includes the electrical supply (28) and the analogue-numerical converter (9).

9. An apparatus according to any of Claims 1 to 8, characterized in that the optical system is completed by one or more mirrors (30, 31, 32) for the simultaneous analysis of two opposite faces of the portion of body (1).

10. An apparatus according to any of Claims 1 to 9, characterized in that the sensor (8) that is illuminated by the reflected beam, and also possibly by a reference beam (11), is a charge-transfer photo-sensor, and more particularly a photo-sensor of the CCD matrix type.

11. An apparatus according to Claim 10, characterized in that means of storing information (39) are inserted between sensor (8) and the analogue-numerical converter proper (9), which delivers to the treatment means (43), « point by point » output signals (41), transformed by an interface (42) for their adaptation to the numerical treatment means (43), monitoring means (44) for the captured data also being provided, in conjunction with the above-mentioned storage means (39).

12. An apparatus according to any of Claims 1 to 11, characterized in that the means of analysis and treatement additionally comprise means to determine and take account of the envelope or volume (46, 47, 50, 51, 53 to 55) within which the prosthesis (45) to be machined should be engraved.

13. An apparatus according to Claim 12, characterized in that the envelope or volume within which the prosthesis (45) to be machined should be engraved is determined, laterally by :

a) the zone of contact (46, 47) between adjacent teeth (48, 49), and

b) planes (50, 51) that are tangential to the arcade of the mouth, or are determined symmetrically, for its vestibular or lingual faces, from below by :

c) the lower limits of the stump (1) analysed in the mouth, as lower plane, and from above by :

d) an upper plane according to a mathematical analysis resulting either from the study of the worn areas of the whole of the maxilla, or of the actual displacement of the mandible.

14. An apparatus according to Claim 12, characterized in that the means of analysis and treatment of signals (43) also comprise automatic means of correction of the theoretical external shape of the prosthesis (45') engraved within the above-mentioned envelope, in accordance with data resulting from the optical analysis, such as the shape of the stump of the tooth (1) in the case where a crown is being produced.

15. An apparatus according to any of Claims 1 to 14, characterized in that the means of analysis and treatment also comprise means to determine static and dynamic occlusion.

16. An apparatus according to Claim, characterized in that the occlusion is determined by datum points on the upper (33) and lower (34) maxillae, separate otpical impressions taken of the two maxillae, creation of an optical grid (57) in front of the datum points of the two maxillae, and bringing the two maxillae together by means of their datum points at grid level.

17. An apparatus according to any of Claims 1 to 16, characterized in that the means of analysis and treatment also comprise means to determine and take account of the colours of shades of colouring of the teeth.

**Patentansprüche**

1. Vorrichtung zum Abnehmen eines Abdrucks mit optischen Mitteln, insbesondere für die automatische Herstellung von Prothesen, vorzugsweise von Prothesen in Form von Kronen für die Zahneilkunde,

mit Strahlungsmitteln (17) zur Aussendung von nicht traumatisierenden Lichtwellen,

mit einem Analysierkopf (13), der Übertragungsmittel (4, 5) umfaßt, durch die die genannten Wellen auf den betreffenden körperteil, z. B. den Ort des zu analysierenden Zahns (1), gerichtet werden können,

mit Empfangsmitteln (6, 7) für die von diesem Körperteil reflektierten Lichtwellen, die die genannten Wellen auf einen Detektor (3) richten, der einem Analog-Digital-Wandler (9) zugeordnet ist, der die Gewinnung einer räumlich dreidimensionalen Darstellung der Form des zu analysierenden Körperteils in Form digitaler Signale ermöglicht,

ferner mit einer Einrichtung (39 bis 44) zur Analyse und Verarbeitung der gewonnenen digitalen Signale, insbesondere im Hinblick auf die automatische Steuerung einer numerisch gesteuerten Werkzeugmaschine (70) zur Bearbeitung einer Prothese, z. B. einer Zahnprothese (45), die exakt an den analysierten Körperteil (1) angepaßt ist,

dadurch gekennzeichnet,

daß die genannten Strahlungsmittel (17) eine Strahlungsquelle für nichtkohärentes Licht umfassen,

und daß die Vorrichtung ferner (an sich bekannte) der Strahlungsquelle (17) bzw. dem Detektor (3) zugeordnete Markierungssraster (19, 19') aufweist, um die Analyse durch holographische Interferometrie zu ermöglichen, sowie Entfernungsbestimmungsmittel, die die Bestimmung oder Festlegung der Entfernung zwischen den optischen Mitteln des Analysierkopfs (13) und einer mit dem zu analysierenden Körperteil (1) verbundenen Referenzebene ermöglichen.

2. Vorrichtung nach Anspruch 1, dadurch gekennzeichnet, daß die Strahlungsmittel (17) Licht in einem Wellenlängenbereich verwenden, das keiner in dem Mund vorhandenen Farbe entspricht, insbesondere indem der Lichtquelle (17) ein Filter zugeordnet wird, das es ermoglicht, beispielsweise im blauen oder grünen Bereich oder zwischen dem blauen und grünen Bereich zu arbeiten,

3. Vorrichtung nach Anspruch 1 oder 2, dadurch gekennzeichnet, daß die dem Analysierkopf (13) zugeordneten Entfernungsbestimmungsmitell aus einem Ultraschall- oder Infrarot-Sendeemfänger bestehen, der die Entfernung des Analysierkopfs (13) von der genannten Referenzebene genau in dem Zeitpunkt bestimmt, in dem Beleuchtung durch die Quelle (17) durchgeführt wird.

4. Vorrichtung nach Anspruch 1 oder 2, dadurch gekennezeichnet, daß die dem Analysierkopf (13) zugeordneten Entfernungsbestimmungsmitel aus einer Markierung (22) bekannter Länge bestehen, die auf einem Punkt des zu analysierenden Körperteils (1) ruhen kann.

5. Vorrichtung nach einem Ansprüche 1 bis 4, dadurch gekennzeichnet, daß die Quelle (17), der Detecktor (8) und der Analog-Digital-Wandler (19) in einem externen Gehäuse (15) angeordnet sind, das über optische Fasern (4, 7) mit dem Analysierkopf (13), z. B. einem in den Mund eines Patienten einführbaren kopf, verbunden sind.

6. Vorrichtung nach Anspruch 5, dadurch gekennzeichnet, daß der Analysierkopf (13) zwei aufnahmelinsen (6', 6'') besitzt, die jeweils an einem Ende der beiden optischen Fasern (7', 7'') befestigt sind, die das reflektierte Strahlenbündel, in zwei zwei Teilbündel unterteilt, weiterleiten, um gleichzeitig zwei entgegengesetzte Seiten des Körperteils (1) zu analysieren.

7. Vorrichtung nach einem der Ansprüche 1 bis 4, dadurch gekennzeichnet, daß sie eine einzige optische Faser (23) enthält, die an ihrem Ende eine Linse (34) trägt, die gleichzeitig das einfallende und das reflektierte Strahlenbündel leitet, wobei letzteres von einem halbdurchlässigen Spiegel (25) auf den dem Wandler zugeordneten Detektor (8) umgelenkt wird.

8. Vorrichtung nach einem der Ansprüche 1 bis 3, dadurch gekennzeichnet, daß der Analysierkopf (13) die Beleuchtungsquelle (17) und den Detektor (8) mit den direkt montierten betreffenden Linsen (5, 6) enthält, und daß der Analysierkopf (13) über elektrische Verbindungen (26, 27) mit einem externen Gehäuse (29) verbunden ist, das die elektrische Speisung (28) und den Analog-Digital-Wandler (9) enthält.

9. Vorrichtung nach einem der Ansprüche 1 bis 8, dadurch gekennzeichnet, daß das optische System durch einen oder mehrere Spiegeln (30, 31, 32) für die gleichzeitige Analyse zweier entgegengesetzter Seiten des Körperteils (1) vervollständigt ist.

10. Vorrichtung nach einem der Ansprüche 1 bis 9, dadurch gekennzeichnet, daß der von dem reflektierten und gegebenenfalls auch einem Referenz-Strahlenbündel (11) beaufschlagte Detektor (8) ein Photosensor mit Ladungskopplung, insbesondere ein CCD-Photosensor in Matrixanordnung ist.

11. Vorrichtung nach Anspruch 10, dadurch gekennzeichnet, daß Informationsspeichermittel (39) zwischen dem Detektor (8) und dem eigentlichen Analog-Digital-Wandler (9) angeordnet sind, der der Verarbeitungseinrichtung (43) « Punkt-für-Punkt »-Ausgangssignale (41) zuführt, die von einem Interface (42) für ihre Anpassung an die dadurch digitale Verarbeitungseinrichtung (43) transformiert werden, und daß Mittel (44) zur visuellen Darstellung der erfaßten Daten vorgesehen sind, die mit den Speichermitteln (39) verbunden sind.

12. Vorrichtung nach einem der Ansprüche 1 bis 11, dadurch gekennzeichnet, daß die Einrichtung zur Analyse und Verarbeitung ferner Mittel (46, 47, 50, 51, 53 bis 55) unfaßt, mit denen sich die Umhüllende oder das Volumen bestimmen und in Rechnung stellen läßt, in das sich die zu bearbeitende Prothese (45) einpassen muß.

13. Vorrichtung nach Anspruch 12, dadurch gekennzeichnet, daß die Umhüllende oder das Volumen, innerhalb dessen sich die zu bearbeitende Prothese (45) einpassen soll, bestimmt wird seitlich durch :

a) die Berührungszonen (46, 47) der Nachbarzähne,

b) für die Vestibular -oder Lingualseiten die die Zahnbogen tangierenden Ebenen (50, 51) oder die auf der Symmetrieseite bestimmten Ebenen, unten durch

c) die Untergrenzen des im Mund analysierten Zahnstumpfs (1) für die untere Ebene, oben durch

d) eine obere Ebene in Abhängigkeit von einer mathematischen Analyse entweder durch Untersuchung der Verschleißzonen der Kiefernanordnung oder der tatsächlichen Verschiebung des Unterkiefers.

14. Vorrichtung nach Anspruch 12, dadurch gekennzeichnet, daß die Analyse - und Verarbeitungseinrichtung (43) der Signale ferner Mittel umfaßt zur automatischen Korrektur der Außenform der in die genannte Umhüllende eingepaßten Prothese, im Fall der Herstellung einer Krone z. B. der Form des Zahnstumpfes (1), in Abhängigkeit von Daten, die sich aus der optischen Analyse ergeben.

15. Vorrichtung nach einem der Ansprüche 1 bis 14, dadurch gekennzeichnet, daß die Analyse- und Verarbeitungseinrichtung ferner Mittel zur Bestimmung des statischen und dynamischen Zahnreihenschlusses umfassen.

16. Vorrichtung nach Anspruch 15, dadurch gekennzeichnet, daß der Zahnreihenschluß bestimmt wird durch Markierung des Ober- und Unterkiefers (33, 34), separate optische Abdrucknahme der beiden Kiefer, Bildung eines vorderen optischen Gitters (57) der Markierungen der beiden Kiefer und Vereinigung der beiden Kiefer dank ihrer Markierungen in Höhe des Gitters.

17. Vorrichtung nach einem der Ansprüche 1 bis 16, dadurch gekennzeichnet, daß die Analyse- und Verarbeitungseinrichtung ferner Mittel zur Bestimmung und Berücksichtigung der Zahnfar-ben und -färbungen umfaßt.

FIG_1

FIG_2

FIG_3

FIG_4

FIG_5

FIG_6

FIG_7

FIG_8

FIG_9

FIG_10

FIG_11

FIG_12

FIG_13

0 091 876

FIG.14

Capteur (photosenseur) 8

Stockage de l'information 39

Carte de mise en oeuvre

Convertisseur analogique numérique 9

Alimentations. spécifiques 40

Sortie numérique (mémoire) des données point par point 41

Visualisation 44

Ordre de Sortie

Calculateur 43

Interface traducteur langage 42

Sortie (Exploitation des données)

4

**0 091 876**

FIG_15

FIG_16

FIG_17

FIG_18

FIG_23

# FIG.19

Capteur

Mise en place des repères buccaux

8

Signal Vidéo

Convertisseur Analog. Numer.

9

Visualisation

44

Maxillaire Supérieur

Maxillaire Inférieur

Claies. Superieures et latérales

Mémoire

Memoire

Mémoire

Assemblage automatique ou manuel des images

Occlusion

# FIG.20

**0 091 876**

FIG. 21

FIG.22

FIG.24

Programme

Information numerique

Visualisation

Données usinage

Usinage

Pupitre

Graphe

0 091 876

FIG. 25

FIG. 26